# EUROPÄISCHE PATENTSCHRIFT

(11) **EP 3 773 226 B1**
(45) Veröffentlichungstag und Bekanntmachung des Hinweises auf die Patenterteilung: **11.05.2022**
(21) Anmeldenummer: 19714634.3
(22) Anmeldetag: 28.03.2019
(51) Int. Cl.: A61B 7/00

(54) **ZWISCHENGLIED FÜR DIE NACHRÜSTUNG EINES MECHANISCHEN STETHOSKOPS**
INTERMEDIATE ELEMENT FOR RETROFITTING A MECHANICAL STETHOSCOPE
ÉLÉMENT INTERMÉDIAIRE POUR L'ÉQUIPEMENT ULTÉRIEUR D'UN STÉTHOSCOPE MÉCANIQUE

(30) Priorität: 13.04.2018 DE 102018205672
(43) Veröffentlichungstag der Anmeldung: 17.02.2021
(73) Patentinhaber: Kirchner & Wilhelm GmbH + Co. KG, 71679 Asperg (DE)
(72) Erfinder: KIRCHNER-GOTTSCHALK, Regina Margarete Clara, 71706 Markgröningen (DE)
(74) Vertreter: Kohler Schmid Möbus Patentanwälte
(86) Internationale Anmeldenummer: PCT/EP2019/057936
(87) Internationale Veröffentlichungsnummer: WO 2019/197169

(56) Entgegenhaltungen:
- US-A- 3 247 324
- US-A- 3 539 724
- US-A- 4 048 444
- US-A1- 2006 098 825
- US-A1- 2015 201 272

## Beschreibung

Die Erfindung betrifft ein Zwischenglied für die Nachrüstung eines mechanischen Stethoskops mit einer elektronischen Verstärkungsfunktion.

Stethoskope dienen dazu, Schallphänomene aus dem Inneren von Körpern besser hörbar zu machen. Insbesondere können die Geräusche von inneren Organen von Patienten, wie Herz oder Lunge, mit einem Stethoskop abgehört werden (so genannte Auskultation) und so Hinweise für den Zustand dieser Organe liefern. Stethoskope können aber auch etwa im Maschinenbau eingesetzt werden, um die Funktion einer Maschine zu überprüfen.

Die verbreitetsten Stethoskope sind mechanische Stethoskope (auch akustische Stethoskope genannt). Bei diesen wird der vom zu untersuchenden Körper aufgenommene Schall von einem Stethoskop-Kopf mechanisch über einen Schlauch bis zu einem Ohrinterface übertragen, an dem der Untersuchende mit seinem Ohr den übertragenen Schall wahrnimmt. Das Ohrinterface umfasst in der Regel eine Ohrolive für ein jeweiliges Ohr des Untersuchenden; die Ohroliven sind typischerweise an den Enden zweier Ohrbügel angeordnet. Der Stethoskop-Kopf weist meist eine am zu untersuchenden Körper aufliegenden Membran auf, oder auch eine Glocke, die dicht am zu untersuchenden Körper aufsitzt. Ein Beispiel eines solchen mechanischen Stethoskops ist das Kircher COLORSCOP^{®} Duo. Mechanische Stethoskope sind vergleichsweise kostengünstig erhältlich und daher sehr verbreitet, sowohl bei Ärzten als auch bei Pflegepersonal.

Mittlerweile werden auch so genannte elektronische Stethoskope eingesetzt. Bei diesen wird der vom zu untersuchenden Körper ausgehende Schall mit einem Mikrofon aufgenommen, elektronisch verstärkt und in verstärkter Form dem Ohr-Interface zugeleitet. Durch die Verstärkung können auch sehr leise Geräusche für eine Auskultation zugänglich gemacht werden. Im Rahmen der elektronischen Verstärkung können zudem spezielle Filterfunktionen eingesetzt werden, etwa um Störungen durch Umgebungsgeräusche zu reduzieren. Ein Beispiel für ein solches elektronisches Stethoskop ist das 3M Littmann^{®} Modell 3200. Elektronische Stethoskope sind bisher vergleichsweise teuer und haben nur eine begrenzte Verbreitung gefunden, insbesondere im medizinischen Bereich. Bei den bekannten elektronischen Stethoskopen ist die Verstärkungsfunktion üblicherweise in den Stethoskop-Kopf integriert.

Eine relevante Problematik stellt zudem die Energieversorgung eines elektronischen Stethoskops dar. Die elektronische Verstärkungsfunktion fällt bei Erschöpfung des Energiespeichers des elektronischen Stethoskops aus; bis zu einer Wiederaufladung oder einem Austausch des Energiespeichers ist das elektronische Stethoskop nicht einsatzbereit. Dies hat - neben den Kosten - die Akzeptanz von elektronischen Stethoskopen begrenzt; viele Ärzte haben lieber auf ein stets einsatzbereites mechanischen Stethoskop zurückgegriffen, um im Notfall keine Verzögerungen bis zum Beginn einer Untersuchung hinnehmen zu müssen.

Aus der WO 2015/105641 A1 ist ein Adapter für ein Stethoskop mit Verstärkungsfunktion bekannt geworden, in welchem eine erste Kammer mit einem Mikrofon und eine zweite Kammer mit einem Lautsprecher ausgebildet ist. Die Kammern sind über ein Umschaltventil miteinander verbunden.

Die US 4,048,444 A beschreibt eine Phonostethoskop-Umwandlungseinheit zur Verstärkung und Verdeutlichung von Körpergeräuschen zur Verwendung mit einem herkömmlichen Stethoskop. Diese elektronische Umwandlungseinheit lässt sich jeweils zwischen einem Ohrbügel und einem abnehmbaren Ohrstöpselstück des Stethoskops anbringen. Die elektronische Umwandlungseinheit umfasst einen Eingangsanschluss, an dem Schall von einem Bruststückelement in einen Schallkanal, der entlang einer Längsachse der Umwandlungseinheit verläuft, gelangt und weiter zu einem Ausgangsanschluss geführt wird. Seitlich am Schallkanal angeordnet sind ein Eingangsmikrofon, welches seine Signale durch eine Vielzahl von Lüftungsschlitzen aus dem Schallkanal empfängt, und ein akustisches Ausgabeelement, welches seine akustische Ausgabe durch eine Vielzahl von Öffnungen in den Schallkanal abgibt. Ein elektronischer Verstärker verstärkt die Signale vom Eingangsmikrofon zum akustischen Ausgabeelement. Durch eine bewegliche Schallwand können der Eingangs- und der Ausgangsanschluss voneinander getrennt werden, um beim elektronisch unterstützten Betrieb ungewollte elektronische Oszillationen auszuschließen.

Die US 3,539,724 A beschreibt ein kombiniert elektronisches und luftsäulen-betriebenes Stethoskop, in welchem von einem Stethoskop-Kopf ein Schlauch zu einem (Eingangs-)Rohr in ein (Verstärker-)Gehäuse führt. Hinter dem Eingangsrohr ist eine Ventileinrichtung mit einem drehbaren Kern angeordnet, durch die Schallwege geschaltet werden können. In einer ersten Drehstellung verbindet eine zentrale Bohrung im Kern das Eingangsrohr mit einem gegenüberliegenden (Ausgangs-)Rohr, an das ein Schlauch angeschlossen ist, der zu den Ohrstücken führt. In einer zweiten Drehstellung wird das Eingangsrohr über eine schräge Bohrung im Kern auf ein (Zwischen-)Rohr geschaltet, das zu einer Konuskammer mit einem Mikrofon führt. Weiterhin ist ein (Zwischen-)Rohr, das von einem Lautsprecher kommt, über einen Durchgang auf das Ausgangsrohr geschaltet.

Aus der US 3,247,324 A, die als nächstliegender Stand der Technik angesehen wird, ist ein akustisches und elektronisches Stethoskop bekannt geworden, in welchem zwischen einem Stethoskop-Kopf und einem Ohrinterface eine Kopplungseinheit angeordnet ist. Schall vom Stethoskop-Kopf wird über einen Schlauch an einem Anschluss in eine erste Kammer geleitet, an dessen Boden ein Mikrofon angeordnet ist. Seitlich von der Kammer weg führt eine mehrfach gebogene, starre Leitung zu einem Drehventil, und vom Drehventil führt eine starre Leitung weiter in eine zweite Kammer. Am Boden der zweiten Kammer ist ein Lautsprecher angeordnet. Über einen Anschluss sind Schläuche zum Ohrinterface angeschlossen.

Aus der US 2006/0098825 A1 ist eine elektronische Adaption eines akustischen Stethoskops bekannt geworden, in welcher zwischen einem Bruststück und einem gegabelten Ohrbügel ein Adapter angeordnet ist, der die detektierten Signale verstärkt und so verarbeitet, dass das ausgegebene Signal verbessert wird.

### Aufgabe der Erfindung

Es ist Aufgabe der Erfindung, eine elektronische Verstärkungsfunktion für Stethoskope auf einfache und kostengünstige Weise nachträglich verfügbar zu machen, und gleichzeitig die Einsatzbereitschaft des Stethoskops zu verbessern, wobei insbesondere in einem elektronischen Verstärkungsbetrieb eine Reduktion von unerwünschten Rückkopplungen erreicht wird, und in einem mechanischen Betrieb eine unbeeinträchtigte Schallleitung ermöglicht wird.

### Beschreibung der Erfindung

Diese Aufgabe wird erfindungsgemäß gelöst durch ein Zwischenglied für ein Stethoskop, wobei das Zwischenglied umfasst
- einen Eingangsanschluss für einen ersten Stethoskop-Teil, der Schall von einen Stethoskop-Kopf heranführt,
- einen Ausgangsanschluss für einen zweiten Stethoskop-Teil, der Schall zu wenigstens einem Ohrinterface führt,
- einen Verbindungskanal zwischen Eingangsanschluss und Ausgangsanschluss, der Schall vom Eingangsanschluss zum Ausgangsanschluss führen kann, und
- eine elektronische Verstärkeranordnung, umfassend
   a) eine Mikrofon-Anordnung zur Aufnahme von über den Eingangsanschluss erhaltenem Schall,
   b) eine Lautsprecher-Anordnung zur Abgabe von Schall zum Ausgangsanschluss,
   c) einen elektronischen Verstärker, mit dem von der Mikrofon-Anordnung aufgenommene Signale verstärkt der Lautsprecher-Anordnung zugeleitet werden können,
   d) einen Energiespeicher zur Versorgung der elektronischen Verstärkeranordnung mit elektrischem Strom,

wobei die Mikrofon-Anordnung und die Lautsprecheranordnung so am Verbindungskanal angeordnet sind, dass die Mikrofon-Anordnung näher zum Eingangsanschluss ist als die Lautsprecher-Anordnung,
wobei die Mikrofon-Anordnung eine Richtcharakteristik aufweist, so dass die Mikrofon-Anordnung von der Seite des Eingangsanschluss ankommenden Schall stärker registriert als von der Seite der Lautsprecher-Anordnung und des Ausgangsanschluss ankommenden Schall,
wobei die Lautsprecher-Anordnung eine Richtcharakteristik aufweist, so dass von der Lautsprecher-Anordnung ausgesandter Schall stärker zur Seite des Ausgangsanschluss geleitet wird als zur Seite der Mikrofon-Anordnung und des Eingangsanschluss,
und wobei eine Verschlusseinrichtung vorhanden ist, die zwischen einer geöffneten Stellung und einer geschlossenen Stellung umschaltbar ist, wobei die Verschlusseinrichtung in der geschlossenen Stellung den Verbindungskanal zwischen der Mikrofon-Anordnung und der Lautsprecheranordnung verschließt, und in der geöffneten Stellung der Verbindungskanal zwischen der Mikrofon-Anordnung und der Lautsprecheranordnung offen ist,
das dadurch gekennzeichnet ist,
dass der Verbindungskanal zwischen Eingangsanschluss und Ausgangsanschluss entlang einer Längsachse verläuft,
und dass zur Mikrofonanordnung und zur Lautsprecheranordnung Richtkanäle gehören, die sowohl in der geöffneten Stellung als auch in der geschlossenen Stellung der Verschlusseinrichtung vom Verbindungskanal abzweigen und schräg zum Verbindungskanal verlaufen.

Die vorliegende Erfindung schlägt ein Zwischenglied (oder Zwischenbauteil) vor, das in ein herkömmliches, mechanisches Stethoskop eingebaut werden kann, insbesondere auch nachträglich.

Mit diesem Zwischenglied kann eine elektronische Verstärkungsfunktion genutzt werden, wenn Bedarf hierzu besteht bzw. der Untersuchende (etwa ein Arzt oder eine Pflegekraft) dies wünscht. Bevorzugt kann mit der Verstärker-Anordnung eine Verstärkung des am Eingangsanschlusses erhaltenen Schalls (Schalldruckpegels) um einen Faktor von wenigstens 3, bevorzugt um einem Faktor von wenigstens 10, erreicht werden.

Es ist aber auch alternativ möglich, das nachgerüstete Stethoskop nach wie vor mechanisch zu nutzten. Hierfür ist im Zwischenglied ein Verbindungskanal vorgesehen, der den Eingangsanschluss und den Ausgangsanschluss miteinander verbindet und daher zwischen diesen Anschlüssen eine mechanische Schallleitung zulässt. Somit ist es insbesondere möglich, bei Erschöpfung des Energiespeichers der elektronischen Verstärkeranordnung das Stethoskop noch zu nutzen (wenngleich nur noch als mechanisches Stethoskop); eine zeitaufwändige Suche nach einem Ersatzstethoskop oder einer Ersatzbatterie ist insbesondere in zeitkritischen Situationen nicht nötig.

Eingangsanschluss und Ausgangsanschluss weisen jeweils eine Öffnung für den Eintritt bzw. Austritt des Schalls auf. Der Verbindungskanal hat typischerweise einen (inneren) Durchmesser DV, der näherungsweise dem (inneren) Durchmesser DST eines jeweiligen schallführenden Abschnitts eines angeschlossenen Stethoskop-Teils entspricht, bevorzugt wobei 1,2^{∗}DST≥DV≥0.8^{∗}DST, so dass der Schall im Wesentlichen unbeeinträchtigt propagieren kann. Der erste Stethoskop-Teil enthält typischerweise einen Stethoskop-Kopf mit einer Membran zur Anlage an den zu untersuchenden Körper und/oder einer Glocke zum Aufsetzen auf den zu untersuchenden Körper. Der zweite Stethoskop-Teil enthält typischerweise wenigstens ein Ohrinterface mit einer Ohrolive für das Ohr oder die Ohren des Untersuchenden. Der Eingangsanschluss und der Ausgangsanschluss ermöglichen bevorzugt eine luftdichte (und in der Regel reversible) Verbindung zu den beiden Stethoskop-Teilen.

Im Rahmen der vorliegenden Erfindung werden die Mikrofon-Anordnung und die Lautsprecher-Anordnung so am Verbindungskanal angeordnet, dass die Mikrofon-Anordnung näher zum Eingangsanschluss ist als die Lautsprecher-Anordnung. Durch die Anordnung der Mikrofon-Anordnung und der Lautsprecher-Anordnung am Verbindungskanal wird dieser im Rahmen der Verstärkungsfunktion mit genutzt. Für unverstärkte als auch für verstärkte Geräusche können im Wesentlichen die gleichen Schallwege im Zwischenglied genutzt werden, was die alternative Auswertung bzw. Auskultation von unverstärkten und verstärkten Geräuschen erleichtert. Die Positionierung der Mikrofon-Anordnung weiter vorne (in Richtung Stethoskop-Kopf) als die Lautsprecher-Anordnung vermindert unerwünschte Rückkopplungen.

Man beachte, dass die Mikrofon-Anordnung eine Richtcharakteristik aufweist, so dass die Mikrofon-Anordnung von der Seite des Eingangsanschluss ankommenden Schall stärker registriert als von der Seite der Lautsprecher-Anordnung und des Ausgangsanschluss ankommenden Schall. Das verbessert die Verstärkung der Geräusche aus dem zu untersuchenden Körper und vermindert den Einfluss von Störungen aus der Umgebung, und reduziert unerwünschte Rückkopplungen weiter.

Die Lautsprecher-Anordnung weist eine Richtcharakteristik auf, so dass von der Lautsprecher-Anordnung ausgesandter Schall stärker zur Seite des Ausgangsanschluss geleitet wird als zur Seite der Mikrofon-Anordnung und des Eingangsanschluss. Auch dies reduziert unerwünschte Rückkopplungen in erheblichem Umfang.

Man beachte, dass zur Mikrofon-Anordnung und zur Lautsprecheranordnung Richtkanäle gehören, die sowohl in der geöffneten Stellung als auch in der geschlossenen Stellung der Verschlusseinrichtung vom Verbindungskanal abzweigen und schräg zum Verbindungskanal verlaufen.

Eine Verschlusseinrichtung ist vorhanden, die zwischen einer geöffneten Stellung und einer geschlossenen Stellung umschaltbar ist, wobei die Verschlusseinrichtung in der geschlossenen Stellung den Verbindungskanal zwischen der Mikrofon-Anordnung und der Lautsprecher-Anordnung verschließt, und in der geöffneten Stellung der Verbindungskanal zwischen der Mikrofon-Anordnung und der Lautsprecher-Anordnung offen ist. Mit der umschaltbaren Verschlusseinrichtung ist es möglich, im elektronischen Verstärkungsbetrieb die Gefahr von Rückkopplungen zwischen Mikrofon-Anordnung und Lautsprecher-Anordnung weiter zu reduzieren; bei geschlossener Stellung der Verschlusseinrichtung wird hierfür die Verbindungskanal für Schall weitgehend blockiert. Um im unverstärkten Fall den Schall ungehindert vom Eingangsanschluss zum Ausgangsanschluss durchleiten zu können, kann die Verschlusseinrichtung in die geöffnete Stellung überführt werden.

Man beachte, dass der Verbindungskanal zwischen Eingangsanschluss und Ausgangsanschluss entlang einer Längsachse verläuft.

Das Zwischenglied ist typischerweise im Wesentlichen zylinderförmig ausgebildet, wobei der Verbindungskanal im Wesentlichen mittig parallel zur Zylinderachse verläuft. Das Zwischenglied ist bevorzugt überwiegend aus Kunststoff gefertigt, um ein geringes Eigengewicht zu erhalten.

### Bevorzugte Ausführungsformen der Erfindung

Bevorzugt ist bei einer Ausführungsform vorgesehen, dass die Verschlusseinrichtung manuell mechanisch umschaltbar ausgebildet ist,
insbesondere wobei die Verschlusseinrichtung eine Verrastung für die geschlossenen Stellung und eine Verrastung für die geöffnete Stellung aufweist. Die manuell mechanische Umschaltung zwischen geöffneter und geschlossener Stellung ist auch problemlos möglich, wenn der Energiespeicher erschöpft sein sollte, und ist zudem kostengünstig und einfach einzurichten. Durch die Verrastung (die meist über einen elastischen Mechanismus eingerichtet ist) ist eine gute Kontrolle über die Stellung der Verschlusseinrichtung möglich.

Bevorzugt ist es auch, wenn die Verschlusseinrichtung mit einer Irisblende ausgebildet ist, insbesondere wobei die Irisblende über einen drehbaren Betätigungsring zwischen der geschlossenen Stellung und der geöffneten Stellung umschaltbar ist. Mittels einer Irisblende kann platzsparend ein Verschluss eines insbesondere zentral im Zwischenglied gelegenen Verbindungskanals erfolgen. Die Umschaltung über einen drehbaren Betätigungsring ist einfach einzurichten.

Vorteilhaft ist weiterhin eine Ausführungsform, bei der der Eingangsanschluss und/oder der Ausgangsanschluss ausgebildet sind mit
- einem Anschlussgewinde und/oder
- einem Steckanschluss, insbesondere einem konisch geformten Steckanschluss. Gewinde und Steckanschlüsse haben sich in der Praxis bewährt und sind einfach und zuverlässig einzurichten, um das erste und/oder zweite Stethoskop-Teil anzuschließen. Konisch geformte Steckanschlüsse gestatten zudem eine gewisse Flexibilität bei den zu verbindenden Steckergrößen, etwa um eine Kompatibilität mit verschiedenen Herstellern von mechanischen Stethoskopen einzurichten. Man beachte, dass an den Anschlussgewinden oder Steckanschlüssen auch Anschlussadapter eingesetzt werden können, um Größenunterschiede auszugleichen.

Ebenfalls bevorzugt ist eine Ausführungsform, bei der der Eingangsanschluss und/oder der Ausgangsanschluss buchsenförmig und/oder rohrstutzenförmig ausgebildet sind, insbesondere wobei der Eingangsanschluss buchsenförmig und der Ausgangsanschluss rohrstutzenförmig ausgebildet sind. Bei herkömmlichen mechanischen Stethoskopen ist meist der Stethoskop-Kopf mit einem rohrstutzenförmigen Steckanschluss ausgebildet, der in einen buchsenförmigen Eingangsanschluss eingebracht werden kann; in einen buchsenförmigen Eingangsanschluss kann zudem auch leicht ein Ohrbügel-Ende eingeführt werden. Auf einen rohrstutzenförmigen Ausgangsanschluss kann leicht ein Schallleitungsschlauch oder eine Ohrolive aufgestülpt werden.

Besonders bevorzugt ist eine Ausführungsform, bei der der elektronische Verstärker wenigstens eine Filterfunktion aufweist, die auf die aufgenommenen Signale angewandt werden kann, insbesondere
- einen Frequenzfilter, bevorzugt einen umschaltbaren Frequenzfilter zur Anpassung an verschiedene Formen eines Stethoskop-Kopfs und/oder einen Frequenzfilter mit mehreren, einzeln regulierbaren Frequenzbereichen, und/oder
- einen Subtraktionsfilter zum Subtrahieren von Umgebungsgeräuschen, insbesondere wobei das Zwischenglied zur Bestimmung von zu subtrahierenden Umgebungsgeräuschen wenigstens ein weiteres Mikrofon aufweist, bevorzugt wobei das wenigstens eine weitere Mikrofon außen am Zwischenglied angeordnet ist. Mit einer Filterfunktion können relevante Informationen aus dem Geräuschen des untersuchten Körpers leichter erkannt bzw. besser wahrnehmbar gemacht werden. Formen des Stethoskop-Kopfs, an die eine Anpassung erfolgen kann, sind insbesondere ein Membran-Typ oder ein Glocken-Typ.

Vorteilhaft ist auch eine Ausführungsform, bei der der Energiespeicher als ein wiederaufladbarer Energiespeicher ausgebildet ist, insbesondere wobei das Zwischenglied eine kabellose Ladeeinrichtung für den Energiespeicher umfasst. Ein wiederaufladbarer Energiespeicher (auch bezeichnet als wiederaufladbare Energiequelle) vermeidet zeitaufwändige Austauschvorgänge. Eine kabellose Ladeeinrichtung ist besonders hygienisch und vermeidet platzaufwendige Steckverbindungen für eine Energieübertragung. Die kabellose Ladeeinrichtung des Zwischenglieds erhält typischerweise Energie über eine induktive Kopplung von einer externen Energiequelle. Als externe Energiequelle für das Zwischenglied kann beispielsweise eine Solar-Ladestation oder eine an das lokale Stromnetz angeschlossene Ladestation eingesetzt werden.

Bevorzugt ist zudem eine Ausführungsform, bei der das Zwischenglied aufweist
- wenigstens eine Eingabevorrichtung zur Einstellung der Verstärkeranordnung; und/oder
- eine Anzeige zur Darstellung eines Patientengeräusches, insbesondere eines Atemgeräusches oder eines Herzschlags, und/oder
- eine elektronische Analyseeinrichtung zur Auswertung eines Patientengeräusches; und/oder

- eine Anzeige zur Darstellung einer aus einem Patientengeräusch abgeleiteten Information, insbesondere einer Herzfrequenz oder einer Atemfrequenz, und/oder
- eine Anzeige zur Darstellung eines verbleibenden Energievorrats des Energiespeichers oder zur Warnung bei Erreichen eines kritischen verbleibenden Energievorrats des Energiespeichers; und/oder
- eine Anzeige zur Darstellung eines Verstärkungsfaktors der elektronischen Verstärkeranordnung; und/oder
- eine Anzeige zur Darstellung einer Frequenzfilter-Einstellung; und/oder
- eine Anzeige zur Darstellung einer Einstellung einer Umgebungsgeräusch-Kompensation; und/oder
- eine drahtlose Datenübertragungsschnittstelle, insbesondere für WLAN oder Bluetooth; und/oder
- eine kabelgebundene Datenübertragungsschnittstelle, insbesondere für USB,
- einen Ein/Aus-Schalter für die elektronische Verstärkungsanordnung. Diese Zusatzfunktionen haben sich in der Praxis bewährt und verbessern den praktischen Nutzen oder erleichtern die Bedienung. Mittels des Ein/Aus-Schalters kann der Energievorrat des Energiespeichers zuverlässig geschont werden.

In den Rahmen der vorliegenden Erfindung fällt auch eine Stethoskop-Anordnung, umfassend
- ein mechanisches Stethoskop mit einem Stethoskop-Kopf, einem Schallübertragungsschlauch, zwei Ohrbügeln und zwei Ohrinterfaces,
- wenigstens ein erfindungsgemäßes, oben beschriebenes Zwischenglied,
wobei das wenigstens eine Zwischenglied zwischen dem Stethoskop-Kopf und den zwei Ohrinterfaces in das mechanische Stethoskop eingebaut ist. Bei dieser Stethoskop-Anordnung kann das Stethoskop wahlweise mit elektronischer Verstärkung oder auch mechanisch betrieben werden. Die Aufrüstung mit dem erfindungsgemäßen Zwischenglied ist sehr leicht möglich.

Ebenfalls in den Rahmen der vorliegenden Erfindung fällt die Verwendung eines erfindungsgemäßen, oben beschriebenen Zwischenglieds zur Nachrüstung eines mechanischen Stethoskops,
wobei das Zwischenglied zwischen einem Stethoskop-Kopf und einem Ohrinterface des Stethoskops eingebaut wird,
wobei ein erster Stethoskop-Teil umfassend den Stethoskop-Kopf am Eingangsanschluss des Zwischenglieds angeschlossen wird, und ein zweiter Stethoskop-Teil umfassend das Ohrinterface am Ausgangsanschluss angeschlossen wird.

Bei der Nachrüstung wird das mechanische Stethoskop typischerweise zwischen erstem Stethoskop-Teil und zweiten Stethoskop-Teil aufgetrennt, und durch das Zwischenglied werden der erste Stethoskop-Teil und der zweite Stethoskop-Teil wieder zu einem ganzen Stethoskop (bzw. einer Stethoskop-Anordnung) verbunden. Die Nachrüstung kann leicht manuell und innerhalb weniger Sekunden erfolgen. Dadurch können vorhandene, mechanische Stethoskope kostengünstig für eine elektronische Verstärkung ertüchtigt werden, um bei Bedarf auch leise oder unauffällige Geräusche einer Untersuchung besser zugänglich zu machen. Gleichzeitig bleiben einfache, mechanische Auskultationen weiterhin problemlos möglich, auch bei etwaigem Strommangel.

Bevorzugt ist eine Variante der erfindungsgemäßen Verwendung, wobei das Zwischenglied am Eingangsanschluss mit oder ohne einen Anschlussadapter an den Stethoskop-Kopf und am Ausgangsanschluss mit oder ohne einen Anschlussadapter an einen Schallübertragungsschlauch angeschlossen wird. An dieser Stelle ist mit einem Zwischenglied eine zentrale Verstärkung für beide Ohrinterfaces möglich.

Bei einer alternativen Variante ist vorgesehen, dass das Zwischenglied am Eingangsanschluss mit oder ohne einen Anschlussadapter an ein Ende eines Ohrbügels und am Ausgangsanschluss mit oder ohne einen Anschlussadapter an eine Ohrolive angeschlossen wird. In dieser Variante wird typischerweise für jedes Ohr ein eigenes Zwischenglied eingesetzt, also insgesamt zwei Zwischenglieder. Bei dieser Bauform werden die von der Lautsprecher-Anordnung bereitgestellten verstärkten Geräusche mit kurzem Weg an das Ohrinterface bzw. in das Ohr des Untersuchenden übertragen, wodurch sich oft Qualitätsgewinne erzielen lassen, insbesondere wenn Filterfunktionen eingesetzt werden.

Weitere Vorteile der Erfindung ergeben sich aus der Beschreibung und der Zeichnung. Ebenso können die vorstehend genannten und die noch weiter ausgeführten Merkmale erfindungsgemäß jeweils einzeln für sich oder zu mehreren in beliebigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt eine schematische Perspektivansicht einer Ausführungsform eines erfindungsgemäßen Zwischenglieds;
- Fig. 2: zeigt eine schematische Querschnittsansicht des Zwischenglieds von Fig. 1, mit der Verschlusseinrichtung in geschlossener Stellung;
- Fig. 3: zeigt eine schematische Querschnittsansicht des Zwischenglieds von Fig. 1, mit der Verschlusseinrichtung in geöffneter Stellung;
- Fig. 4: zeigt die erfindungsgemäße Verwendung eines Zwischengliedes bei der Nachrüstung eines mechanischen Stethoskops in einer ersten Variante, mit (a) das mechanische Stethoskop vor der Nachrüstung; mit (b) Auftrennen des mechanischen Stethoskops zwischen Stethoskop-Kopf und Schallübertragungsschlauch, und mit (c) das mechanische Stethoskop mit eingebautem Zwischenglied;
- Fig. 5: zeigt die erfindungsgemäße Verwendung zweier Zwischenglieder bei der Nachrüstung eines mechanischen Stethoskops in einer zweiten Variante, mit (a) das mechanische Stethoskop vor der Nachrüstung; mit (b) Auftrennen des mechanischen Stethoskops zwischen den Ohrbügel-Enden und den Ohroliven, und mit (c) das mechanische Stethoskop mit zwei eingebauten Zwischengliedern.

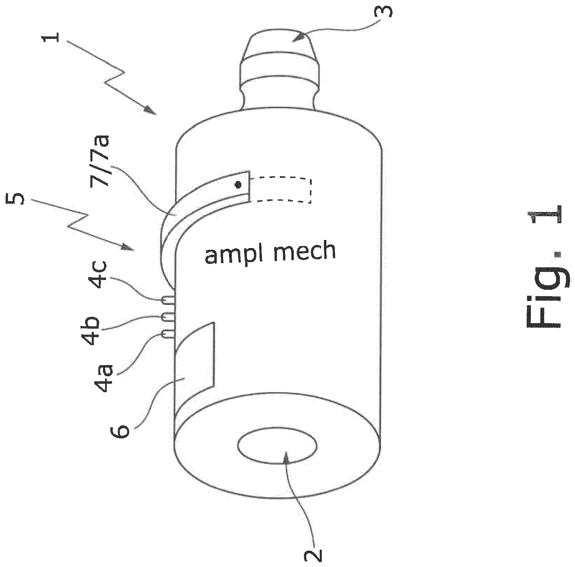

Die **Fig. 1** zeigt in einer schematischen Perspektivansicht ein erfindungsgemäßes Zwischenglied 1, das für die Nachrüstung eines mechanischen Stethoskops mit einer elektronischen Verstärkungsfunktion gemäß der Erfindung eingesetzt werden kann.

Das Zwischenglied 1 ist in der gezeigten Ausführungsform im Wesentlichen kreiszylinderförmig aufgebaut. An seinem in Fig. 1 linkseitigen Ende verfügt das Zwischenglied 1 über einen Eingangsanschluss 2, der hier buchsenförmig mit einer zugehörigen Öffnung für eine Steckverbindung zu einem ersten Stethoskop-Teil ausgebildet ist. An seinem in Fig. 1 rechtsseitigen Ende verfügt das Zwischenglied über einen Ausgangsanschluss 3, der hier rohrstutzenförmig mit einer entsprechenden Öffnung (in Fig. 1 verdeckt) für eine Steckverbindung zu einem zweiten Stethoskop-Teil ausgebildet ist. Über den Eingangsanschluss 2 kann Schall, etwa Geräusche aus dem Körper eines Patienten, in das Zwischenglied 1 eingeleitet werden. Dieser Schall kann wahlweise unverstärkt durch das Zwischenglied 1 zum Ausgangsanschluss 3 durchgeleitet werden, oder auch durch eine elektronische Verstärkeranordnung 5 im Zwischenglied 1 verstärkt werden, wobei dann der verstärkte Schall dem Ausgangsanschluss 3 zugeleitet wird.

Am Zwischenglied 1 sind mehrere Bedienknöpfe (Eingabevorrichtungen) 4a-4c ausgebildet, mit denen die Verstärkeranordnung 5 eingestellt und betrieben werden kann; einer dieser Bedienknöpfe 4a dient auch als ein Ein/Aus-Schalter. Über die Bedienknöpfe 4a-4c kann beispielsweise der Verstärkungsfaktor ("Lautstärke") eingestellt werden, oder auch eine Filterfunktion vorgewählt werden, mit der beispielsweise Umgebungsgeräusche reduziert werden können. Über eine flächige Anzeige (Display) 6, etwa ein LCD-Display, können die momentanen Einstellungen der Verstärkeranordnung 5 abgelesen werden, oder auch registrierte Patientengeräusche grafisch dargestellt werden, oder auch eine automatisch gemessene Herzfrequenz angezeigt werden.

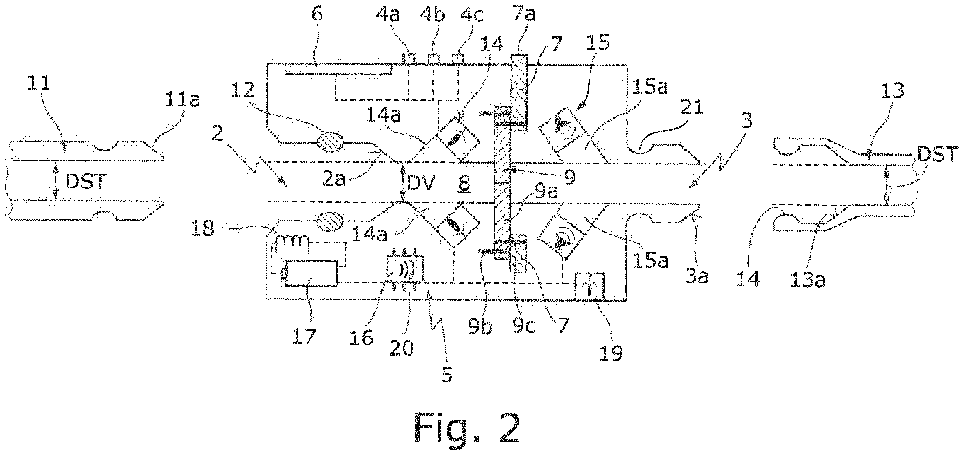

Weiterhin ist am Zwischenglied 1 ein Betätigungsring 7 (großteils verdeckt in Fig. 1) mit einem Betätigungselement 7a ausgebildet, mit dem eine Verschlusseinrichtung geöffnet und geschlossen werden kann (siehe dazu Fig. 2). Mit dem Betätigungselement 7a kann der Betätigungsring 7 zwischen einer Drehposition für den Verstärkungsbetrieb ("ampl") und einer Drehposition für den unverstärkten, mechanischen Betrieb ("mech") manuell verstellt werden.

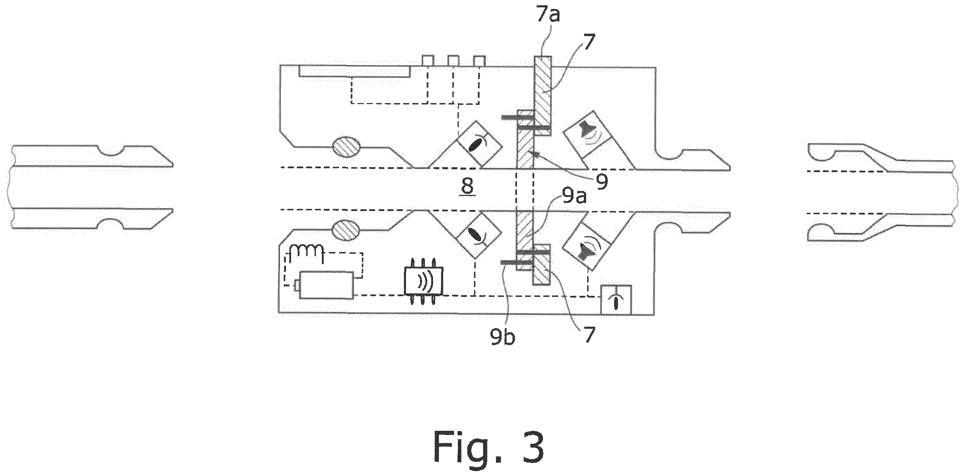

Die **Fig. 2** und die **Fig. 3** zeigen im schematischen Querschnitt den inneren Aufbau des Zwischenglieds 1; in der Fig. 2 ist dabei ein innen liegender Verbindungskanal 8 zwischen dem Eingangsanschluss 2 und dem Ausgangsanschluss 3 mit einer Verschlusseinrichtung 9 blockiert, wohingegen in Fig. 3 der Verbindungskanal 8 bei zurückgezogener Verschlusseinrichtung 9 geöffnet ist.

In den büchsenförmigen Eingangsanschluss 2, der als konisch geformter Steckanschluss mit einer Konusfläche 2a ausgebildet ist, kann ein rohrstutzenartiger, ebenfalls konisch geformter Steckanschluss eines ersten Stethoskop-Teils 11 mit einer Konusfläche 11a eingeführt werden. Mit einem O-Ring 12 kann dabei eine luftdichte Abdichtung erzielt werden.

Am rohrstutzenförmigen Ausgangsanschluss 3, der als konisch geformter Steckanschluss mit einer Konusfläche 3a ausgebildet ist, kann ein buchsenartiger, ebenfalls konisch geformter Steckanschluss eines zweiten Stethoskop-Teils 13 mit einer Konusfläche 13a aufgesetzt werden. Das Material des anschließenden zweiten Stethoskop-Teils ist hier gummielastisch ausgebildet, und ein radial nach innen vorstehender Wulst kann in eine Vertiefung 21 am Ausgangsanschluss 3 eingreifen, um auch auf dieser Seite einen luftdichten Abschluss zu erzielen.

Die inneren Durchmesser DST der schalleitenden Abschnitte in den Stethoskop-Teilen 11, 13 entsprechen hier dem inneren Durchmesser DV im Verbindungskanal 8; im eingesteckten/aufgesteckten Zustand (gestrichelt angedeutet) der Stethoskop-Teile 11, 13 gibt es keine merklichen Stufen im Bereich der inneren Wand des Verbindungskanals 8 oder der Übergänge zu den Stethoskop-Teilen 11, 13.

Jedoch zweigen vom Verbindungskanal 8 in der gezeigten Ausführungsform jeweils zwei Richtkanäle 14a ab, die zu den beiden Mikrofonen einer Mikrofon-Anordnung 14 gehören. Die Richtkanäle 14a verlaufen hier gesehen von der Seite des Eingangsanschlusses 2 in einem Winkel von ca. 45° gegenüber der Achse (Längsachse) des Verbindungskanals 8, so dass von der Eingangsanschluss-Seite her vordringender Schall leicht zu den Mikrofonen der Mikrofon-Anordnung 14 gelangen kann, nicht aber etwaiger von der Ausgangsanschluss-Seite her vordringender Schall.

Weiterhin zweigen vom Verbindungskanal 8 in der gezeigten Ausführungsform jeweils zwei weitere Richtkanäle 15a ab, die zu den beiden Lautsprechern einer Lautsprecher-Anordnung 15 gehören. Die Richtkanäle 15a verlaufen hier gesehen von der Seite des Eingangsanschlusses 2 in einem Winkel von ca. 135° gegenüber der Achse des Verbindungskanals 8, so dass von den Lautsprechern ausgesandeter Schall leicht zum Ausgangsanschluss 3 gelangen kann, nicht aber zu den Mikrofonen der Mikrofon-Anordnung 14.

Zwischen der Mikrofon-Anordnung 14 und der Lautsprecher-Anordnung 15 kann der Verbindungskanal 8 mit der Verschlusseinrichtung 9 blockiert werden. Die Verschlusseinrichtung 9 ist hier mit einer Irisblende ausgebildet, die mehrere Blendenelemente 9a umfasst. Die Blendenelemente 9a sind über fixe Achsstifte 9b am Zwischenglied 1 beweglich gelagert; die fixen Achsstifte 9b sind dabei ringförmig um den Verbindungskanal 8 verteilt. In die Blendenelemente 9a greifen zudem noch bewegliche Führungsstifte 9c ein, die ihrerseits am Betätigungsring 7 gelagert sind. Durch Drehen des Betätigungsrings (um eine Achse, die parallel zum Verbindungskanal 8 verläuft) kann die Schwenkstellung der Blendenelemente 9a um die fixen Achsstifte 9b verändert werden, und dadurch die Öffnungsweite der Irisblende verändert werden.

In der in Fig. 2 gezeigten Stellung ist die Irisblende der Verschlusseinrichtung 9 vollständig geschlossen, und die mechanische Schallleitung durch den Verbindungskanal 8 ist minimiert. Diese Stellung ist für den elektronischen Verstärkungsbetrieb optimal.

In der in Fig. 3 gezeigten Stellung ist die Irisblende so weit geöffnet, dass ihre Öffnungsweite dem inneren Durchmesser DV des (übrigen) Verbindungskanals 8 entspricht; die Schalleitung durch den Verbindungskanal 8 ist unbeeinträchtigt. Diese Stellung ist für den mechanischen (unverstärkten) Betrieb optimal.

Im elektronischen Verstärkungsbetrieb wird die elektronische Verstärkeranordnung 5 betrieben. Über die Mikrofon-Anordnung 14 wird vom Eingangsanschluss 2 ankommender Schall (enthaltend Patientengeräusche) registriert, im elektronischen Verstärker 16 verstärkt und über die Lautsprecher-Anordnung 15 verstärkt wiedergegeben und so dem Ausgangsanschluss 3 zugeleitet. Die Stromversorgung der Verstärkeranordnung 5 stellt dabei ein Energiespeicher 17 sicher, hier ein wiederaufladbarer Akku. Der Energiespeicher 17 ist über eine kabellose Ladevorrichtung 18, die an einer Induktionsspule Energie aus einem externen elektromagnetischen Feld aufnehmen kann, aufladbar; der Ladezustand des Energiespeichers 17 kann an der Anzeige 6 angegeben werden. Der Verstärker 16 ist hier integral mit einer elektronischen Steuer- und Auswerteeinrichtung, mit der Befehle von den Bedienknöpfen 4a-4c entgegengenommen werden können, oder auch die Anzeige 6 angesteuert werden kann, und auch die aufgenommenen Signale aufbereitet oder ausgewertet werden können. Insbesondere kann der Verstärker 16 von einem weiteren Mikrofon 19 Umgebungsgeräusch-Informationen erhalten, so dass bei der Verstärkung der an der Mikrofon-Anordnung 14 erhaltener Geräusche Anteile von Umgebungsgeräuschen in Abzug gebracht werden können, so dass gezielt vor allem Patientengeräusche (und eben nicht Umgebungsgeräusche) verstärkt werden können. Das weitere Mikrofon 19 ist an der Außenseite des Zwischenglieds 1 angeordnet ("Außenmikrofon"). In der gezeigten Ausführungsform verfügt der Verstärker 16 zudem über eine WLAN-Anbindung 20, um beispielsweise aufgenommene Patientengeräusche an einen externen Computer kabellos zu übertragen.

Im mechanischen Betrieb wird die Verstärkungsfunktion über vollständig deaktiviert, wobei die Verstärkeranordnung über den Bedienknopf 4a ausgeschaltet wird.

Optional kann das Zwischenglied 1 mit einem GPS-Empfänger (nicht näher dargestellt) ausgerüstet werden, mit dem der Aufenthaltsort des Zwischenglieds 1 ermittelt und an einen externen Empfänger übermittelt werden kann. Dadurch kann das Zwischenglied 1 leicht wiedergefunden werden, etwa im Falle eines Diebstahls.

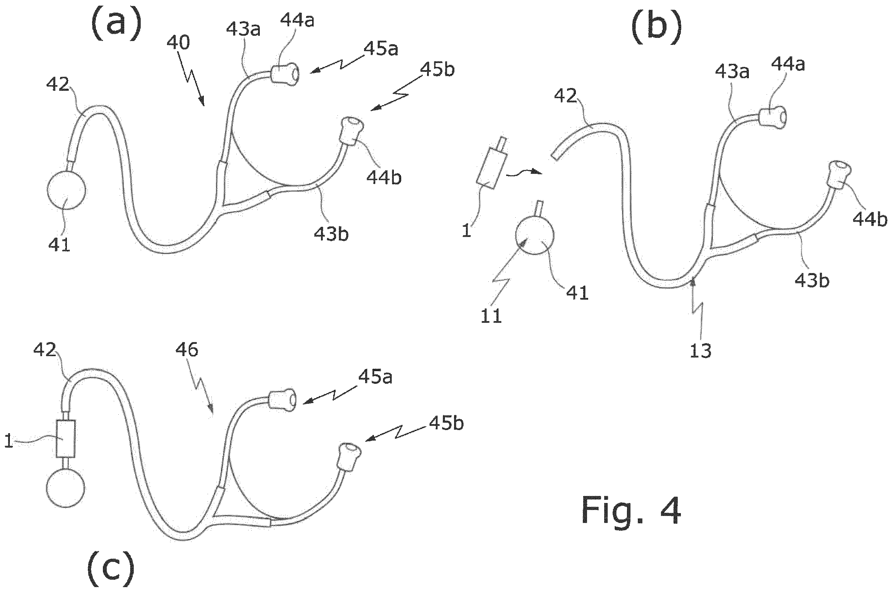

Die **Fig. 4** zeigt die Nachrüstung eines mechanischen Stethoskops 40 in einer ersten Variante für die Erfindung.

In Teilabbildung (a) ist zunächst ein herkömmliches mechanisches Stethoskop 40 zu sehen, welches im Rahmen der Erfindung nachgerüstet werden kann. Das mechanische Stethoskop 40 verfügt über einen Stethoskop-Kopf 41, an den ein Schallübertragungsschlauch 42 angeschlossen ist. In der gezeigten Ausführungsform verzweigt der Schallübertragungsschlauch 42 auf zwei Ohrbügel 43a, 43b (in anderen Ausführungsformen kann der Schallübertragungsschlauch auch beispielsweise an einem Verteilerstück enden, das seinerseits an zwei Ohrbügel angeschlossen ist). Am Ende eines jeweiligen Ohrbügels 43a, 43b ist als Ohrinterface 45a, 45b jeweils eine Ohrolive 44a, 44b aufgesteckt. Die Ohroliven 44a, 44b werden von einem Untersuchenden in einen vorderen Teil des Gehörgangs seiner beiden Ohren eingehängt.

Der Stethoskop-Kopf (auch Bruststück genannt) 41 kann insbesondere vom Membran-Typ (auch "Flachkopf-Stethoskop" genannt) oder vom Glockentyp (auch "Trichter-Stethoskop" genannt) sein, oder auch eine Kombination beider Typen (auch "Doppelkopf-Stethoskop" genannt).

Für die Nachrüstung des mechanischen Stethoskops 40 ist in der ersten Variante vorgesehen, das Stethoskop 40 zwischen Stethoskop-Kopf 41 und Schallübertragungsschlauch 42 aufzutrennen, vgl. Abbildung (b). Der Stethoskop-Kopf 41 bildet dann einen ersten Stethoskop-Teil 11, und das übrige Stethoskop (mit Schallübertragungsschlauch 42, Ohrbügeln 43a, 43b und Ohroliven 44a, 44b) bildet einen zweiten Stethoskop-Teil 13. Zwischen erstem Stethoskop-Teil 11 und zweitem Stethoskop-Teil 13 wird ein Zwischenglied 1 eingefügt. Dabei wird der Stethoskop-Kopf 41 am Eingangsanschluss angeschlossen (hier eingesteckt), und der Schallübertragungsschlauch 42 wird am Ausgangsanschluss angeschlossen (hier aufgesteckt).

Dadurch wird eine Stethoskop-Anordnung 46 erhalten, vgl. Abbildung (c), die sowohl mechanisch (unverstärkt) als auch mit elektronischer Verstärkung zum Abhören eines Patienten eingesetzt werden kann. Verstärkter Schall wird hier vom Zwischenglied 1 in den Schallübertragungsschlauch 42 abgegeben, wobei sich der verstärkte Schall auf beide Ohrinterfaces 45a, 45b verteilt.

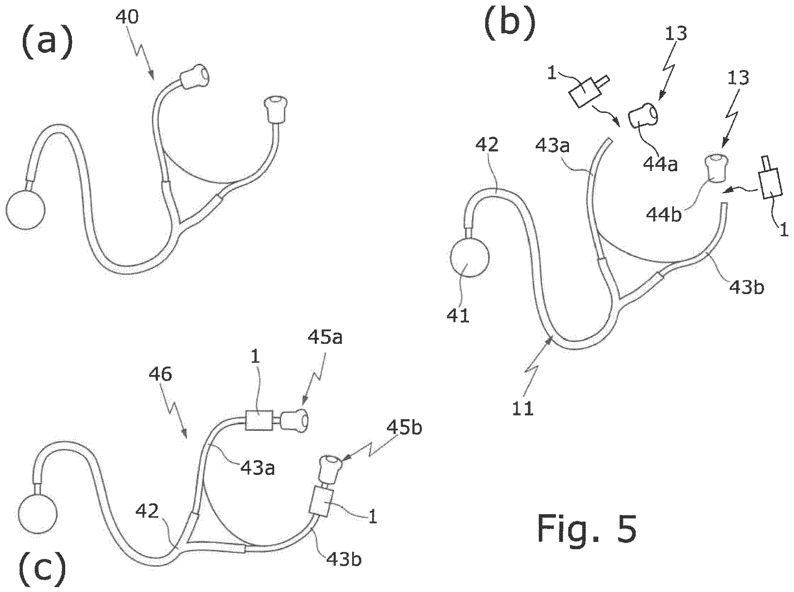

Die **Fig. 5** zeigt die Nachrüstung eines mechanischen Stethoskops 40 in einer zweiten Variante für die Erfindung.

Abbildung (a) zeigt zunächst wieder das mechanische Stethoskop 40 vor der Nachrüstung.

Für die Nachrüstung des mechanischen Stethoskops 40 ist in der zweiten Variante vorgesehen, das Stethoskop 40 zwischen den Enden der Ohrbügel 43a, 43b und den Ohroliven 44a, 44b aufzutrennen, vgl. Abbildung (b). Die Ohroliven 44a, 44b bilden jeweils einen zweiten Stethoskop-Teil 13, und das übrige Stethoskop (mit Stethoskop-Kopf 41, Schallübertragungsschlauch 42 und Ohrbügeln 43a, 43b) bildet einen ersten Stethoskop-Teil 11. Zwischen erstem Stethoskop-Teil 11 und jeweiligem zweitem Stethoskop-Teil 13 wird jeweils ein Zwischenglied 1 eingefügt; insgesamt werden hier also zwei separate Zwischenglieder 1 in das mechanische Stethoskop 40 eingefügt. Dabei wird der jeweilige Ohrbügel 43a, 43b am Eingangsanschluss angeschlossen (hier eingesteckt), und die jeweilige Ohrolive 44a, 44b wird am Ausgangsanschluss angeschlossen (hier aufgesteckt).

Dadurch wird eine Stethoskop-Anordnung 46 erhalten, vgl. Abbildung (c), die sowohl mechanisch (unverstärkt) als auch mit elektronischer Verstärkung zum Abhören eines Patienten eingesetzt werden kann. Unverstärkter Schall wird hier über den verzweigten Schallübertragungsschlauch 42 auf die Ohrbügel 43a, 43b verteilt, und ein jeweiliger Teil des unverstärkten Schalls wird dann von einem der Zwischenglieder 1 verstärkt und der jeweilige verstärkte Schall am jeweiligen Ohrinterface 45a, 45b zur Verfügung gestellt.

### Bezugszeichenliste

- 1: Zwischenglied
- 2: Eingangsanschluss
- 3: Ausgangsanschluss
- 4a: Bedienknopf / Eingabevorrichtung / Ein-/Ausschalter
- 4b-c: Bedienknopf / Eingabevorrichtung
- 5: elektronische Verstärkeranordnung
- 6: Display
- 7: Betätigungsring
- 7a: Betätigungselement
- 8: Verbindungskanal
- 9: Verschlusseinrichtung
- 9a: Blendenelement
- 9b: Achsstift
- 9c: Führungsstift
- 11: erster Stethoskop-Teil
- 11a: Konusfläche
- 12: O-Ring
- 13: zweiter Stethoskop-Teil
- 13a: Konusfläche
- 14: Mikrofon-Anordnung
- 14a: Richtkanal
- 15: Lautsprecher-Anordnung
- 15a: Richtkanal
- 16: elektronischer Verstärker
- 17: Energiespeicher (hier Akku)
- 18: kabellose Ladevorrichtung
- 19: weiteres Mikrofon (Außenmikrofon)
- 20: WLAN-Anbindung
- 21: Vertiefung
- 40: mechanisches Stethoskop für Nachrüstung
- 41: Stethoskop-Kopf
- 42: Schallübertragungsschlauch
- 43a-b: Ohrbügel
- 44a-b: Ohrolive
- 45a-b: Ohrinterface
- 46: Stethoskop-Anordnung
- DST: (innerer) Durchmesser im ersten/zweiten Stethoskop-Teil
- DV: (innerer) Durchmesser im Verbindungskanal

## Patentansprüche

1. Zwischenglied (1) für ein Stethoskop (40), wobei das Zwischenglied (1) umfasst
- einen Eingangsanschluss (2) für einen ersten Stethoskop-Teil (11), der Schall von einen Stethoskop-Kopf (41) heranführt,
- einen Ausgangsanschluss (3) für einen zweiten Stethoskop-Teil (13), der Schall zu wenigstens einem Ohrinterface (45a-b) führt,
- einen Verbindungskanal (8) zwischen Eingangsanschluss (2) und Ausgangsanschluss (3), der Schall vom Eingangsanschluss (2) zum Ausgangsanschluss (3) führen kann, und
- eine elektronische Verstärkeranordnung (5), umfassend
a) eine Mikrofon-Anordnung (14) zur Aufnahme von über den Eingangsanschluss (2) erhaltenem Schall,
b) eine Lautsprecher-Anordnung (15) zur Abgabe von Schall zum Ausgangsanschluss (3),
c) einen elektronischen Verstärker (16), mit dem von der Mikrofon-Anordnung (14) aufgenommene Signale verstärkt der Lautsprecher-Anordnung (15) zugeleitet werden können,
d) einen Energiespeicher (17) zur Versorgung der elektronischen Verstärkeranordnung (5) mit elektrischem Strom;
wobei die Mikrofon-Anordnung (14) und die Lautsprecher-Anordnung (15) so am Verbindungskanal (8) angeordnet sind, dass die Mikrofon-Anordnung (14) näher zum Eingangsanschluss (2) ist als die Lautsprecher-Anordnung (3),
wobei die Mikrofon-Anordnung (14) eine Richtcharakteristik aufweist, so dass die Mikrofon-Anordnung (14) von der Seite des Eingangsanschluss (2) ankommenden Schall stärker registriert als von der Seite der Lautsprecher-Anordnung (15) und des Ausgangsanschluss (3) ankommenden Schall,
wobei die Lautsprecher-Anordnung (15) eine Richtcharakteristik aufweist, so dass von der Lautsprecher-Anordnung (15) ausgesandter Schall stärker zur Seite des Ausgangsanschluss (3) geleitet wird als zur Seite der Mikrofon-Anordnung (14) und des Eingangsanschluss (2),
und wobei eine Verschlusseinrichtung (9) vorhanden ist, die zwischen einer geöffneten Stellung und einer geschlossenen Stellung umschaltbar ist, wobei die Verschlusseinrichtung (9) in der geschlossenen Stellung den Verbindungskanal (8) zwischen der Mikrofon-Anordnung (14) und der Lautsprecher-Anordnung (15) verschließt, und in der geöffneten Stellung der Verbindungskanal (8) zwischen der Mikrofon-Anordnung (14) und der Lautsprecher-Anordnung (15) offen ist,
**dadurch gekennzeichnet,**
**dass** der Verbindungskanal (8) zwischen Eingangsanschluss (2) und Ausgangsanschluss (3) entlang einer Längsachse verläuft,
und **dass** zur Mikrofon-Anordnung (14) und zur Lautsprecheranordnung (15) Richtkanäle (14a, 15a) gehören, die sowohl in der geöffneten Stellung als auch in der geschlossenen Stellung der Verschlusseinrichtung (9) vom Verbindungskanal (8) abzweigen und schräg zum Verbindungskanal (8) verlaufen.

2. Zwischenglied (1) nach Anspruch 1, **dadurch gekennzeichnet,**
**dass** die Verschlusseinrichtung (9) manuell mechanisch umschaltbar ausgebildet ist,
insbesondere wobei die Verschlusseinrichtung (9) eine Verrastung für die geschlossenen Stellung und eine Verrastung für die geöffnete Stellung aufweist.

3. Zwischenglied (1) nach Anspruch 1 oder 2, **dadurch gekennzeichnet,**
**dass** die Verschlusseinrichtung (9) mit einer Irisblende ausgebildet ist.

4. Zwischenglied (1) nach Anspruch 3, **dadurch gekennzeichnet, dass** die Irisblende über einen drehbaren Betätigungsring (7) zwischen der geschlossenen Stellung und der geöffneten Stellung umschaltbar ist.

5. Zwischenglied (1) nach Anspruch 4, **dadurch gekennzeichnet,**
**dass** der drehbare Betätigungsring (7) mit einem Betätigungselement (7a) ausgebildet ist, mit dem der Betätigungsring (7) zwischen einer Drehposition für einen Verstärkungsbetrieb und einer Drehposition für einen mechanischen, unverstärkten Betrieb manuell verstellt werden kann, insbesondere wobei das Betätigungselement (7a) radial gegenüber dem übrigen Zwischenglied (1) hervorsteht.

6. Zwischenglied (1) nach einem der vorhergehenden Ansprüche, **dadurch gekennzeichnet, dass** der Eingangsanschluss (2) und/oder der Ausgangsanschluss (3) ausgebildet sind mit
- einem Anschlussgewinde und/oder
- einem Steckanschluss, insbesondere einem konisch geformten Steckanschluss.

7. Zwischenglied (1) nach einem der vorhergehenden Ansprüche, **dadurch gekennzeichnet, dass** der Eingangsanschluss (2) und/oder der Ausgangsanschluss (3) buchsenförmig und/oder rohrstutzenförmig ausgebildet sind.

8. Zwischenglied nach Anspruch 7, **dadurch gekennzeichnet, dass** der Eingangsanschluss (2) buchsenförmig und der Ausgangsanschluss (3) rohrstutzenförmig ausgebildet sind.

9. Zwischenglied (1) nach einem der vorhergehenden Ansprüche, **dadurch gekennzeichnet, dass** der elektronische Verstärker (16) wenigstens eine Filterfunktion aufweist, die auf die aufgenommenen Signale angewandt werden kann, insbesondere
- einen Frequenzfilter, bevorzugt einen umschaltbaren Frequenzfilter zur Anpassung an verschiedene Formen eines Stethoskop-Kopfs (21) und/oder einen Frequenzfilter mit mehreren, einzeln regulierbaren Frequenzbereichen, und/oder
- einen Subtraktionsfilter zum Subtrahieren von Umgebungsgeräuschen, insbesondere wobei das Zwischenglied (1) zur Bestimmung von zu subtrahierenden Umgebungsgeräuschen wenigstens ein weiteres Mikrofon (19) aufweist, bevorzugt wobei das wenigstens eine weitere Mikrofon (19) außen am Zwischenglied (1) angeordnet ist.

10. Zwischenglied (1) nach einem der vorhergehenden Ansprüche, **dadurch gekennzeichnet, dass** der Energiespeicher (17) als ein wiederaufladbarer Energiespeicher (17) ausgebildet ist, insbesondere wobei das Zwischenglied (1) eine kabellose Ladeeinrichtung (18) für den Energiespeicher (17) umfasst.

11. Zwischenglied (1) nach einem der vorhergehenden Ansprüche, **dadurch gekennzeichnet, dass** das Zwischenglied (1) aufweist
- wenigstens eine Eingabevorrichtung (4a-c) zur Einstellung der Verstärkeranordnung (5); und/oder
- eine Anzeige (6) zur Darstellung eines Patientengeräusches, insbesondere eines Atemgeräusches oder eines Herzschlags, und/oder
- eine elektronische Analyseeinrichtung zur Auswertung eines Patientengeräusches; und/oder
- eine Anzeige (6) zur Darstellung einer aus einem Patientengeräusch abgeleiteten Information, insbesondere einer Herzfrequenz oder einer Atemfrequenz, und/oder
- eine Anzeige (6) zur Darstellung eines verbleibenden Energievorrats des Energiespeichers (17) oder zur Warnung bei Erreichen eines kritischen verbleibenden Energievorrats des Energiespeichers (17); und/oder
- eine Anzeige (6) zur Darstellung eines Verstärkungsfaktors der elektronischen Verstärkeranordnung (5); und/oder
- eine Anzeige (6) zur Darstellung einer Frequenzfilter-Einstellung; und/oder
- eine Anzeige (6) zur Darstellung einer Einstellung einer Umgebungsgeräusch-Kompensation; und/oder
- eine drahtlose Datenübertragungsschnittstelle, insbesondere für WLAN (20) oder Bluetooth; und/oder
- eine kabelgebundene Datenübertragungsschnittstelle, insbesondere für USB,
- einen Ein/Aus-Schalter (4a) für die elektronische Verstärkungsanordnung (5) .

12. Zwischenglied (1) nach einem der vorhergehenden Ansprüche, **dadurch gekennzeichnet,**
**dass** das Zwischenglied (1) überwiegend aus Kunststoff gefertigt ist, insbesondere aus einem schallisolierenden Kunststoff.

13. Zwischenglied (1) nach einem der vorhergehenden Ansprüche, **dadurch gekennzeichnet, dass** das Zwischenglied (1) eine Außenhülle aus Kunststoff aufweist, insbesondere aus einem schallisolierenden und/oder gummielastischen Kunststoff.

14. Zwischenglied (1) nach einem der vorhergehenden Ansprüche, **dadurch gekennzeichnet,**
**dass** der elektronische Verstärker (16) wenigstens eine Filterfunktion aufweist, die auf die aufgenommenen Signale angewandt werden kann, nämlich einen Subtraktionsfilter zum Subtrahieren von Umgebungsgeräuschen, und das Zwischenglied (1) zur Bestimmung von zu subtrahierenden Umgebungsgeräuschen wenigstens ein weiteres Mikrofon (19) aufweist, das außen am Zwischenglied (1) angeordnet ist.

15. Zwischenglied (1) nach einem der vorhergehenden Ansprüche, **dadurch gekennzeichnet, dass** die Richtkanäle (14a, 15a) unter einem Winkel von ca. 45° und unter einem Winkel von ca. 135° gegenüber der Längsachse des Verbindungskanals (8) verlaufen.

16. Stethoskop-Anordnung (46), umfassend
- ein mechanisches Stethoskop (40) mit einem Stethoskop-Kopf (41), einem Schallübertragungsschlauch (42), zwei Ohrbügeln (43a-b) und zwei Ohrinterfaces (45a-b),
- wenigstens ein Zwischenglied (1) nach einem der Ansprüche 1 bis 15, wobei das wenigstens eine Zwischenglied (1) zwischen dem Stethoskop-Kopf (41) und den zwei Ohrinterfaces (45a-b) in das mechanische Stethoskop (40) eingebaut ist.

17. Verwendung eines Zwischenglieds (1) nach einem der Ansprüche 1 bis zur Nachrüstung eines mechanischen Stethoskops (40),
wobei das Zwischenglied (1) zwischen einem Stethoskop-Kopf (41) und einem Ohrinterface (45a-b) des Stethoskops (40) eingebaut wird,
wobei ein erster Stethoskop-Teil (11) umfassend den Stethoskop-Kopf (41) am Eingangsanschluss (2) des Zwischenglieds (1) angeschlossen wird, und ein zweiter Stethoskop-Teil (13) umfassend das Ohrinterface (45a-b) am Ausgangsanschluss (3) angeschlossen wird.

18. Verwendung nach Anspruch 17, **dadurch gekennzeichnet, dass** das Zwischenglied (1) am Eingangsanschluss (2) mit oder ohne einen Anschlussadapter an den Stethoskop-Kopf (41) und am Ausgangsanschluss (3) mit oder ohne einen Anschlussadapter an einen Schallübertragungsschlauch (42) angeschlossen wird.

19. Verwendung nach Anspruch 17, **dadurch gekennzeichnet, dass** das Zwischenglied (1) am Eingangsanschluss (2) mit oder ohne einen Anschlussadapter an ein Ende eines Ohrbügels (43a-b) und am Ausgangsanschluss (3) mit oder ohne einen Anschlussadapter an eine Ohrolive (44a-b) angeschlossen wird.

## Claims

1. Intermediate member (1) for a stethoscope (40), wherein the intermediate member (1) comprises
- an input connection (2) for a first stethoscope part (11), which introduces sound from a stethoscope head (41),
- an output connection (3) for a second stethoscope part (13), which leads sound to at least one ear interface (45a-b),
- a connecting channel (8) between the input connection (2) and the output connection (3), which can lead sound from the input connection (2) to the output connection (3), and
- an electronic amplifier arrangement (5), comprising
a) a microphone arrangement (14) for recording sound received via the input connection (2),
b) a loudspeaker arrangement (15) for delivering sound to the output connection (3),
c) an electronic amplifier (16) with which signals picked up by the microphone arrangement (14) can be amplified and fed to the loudspeaker arrangement (15),
d) an energy storage (17) for supplying the electronic amplifier arrangement (5) with electric power,
wherein the microphone arrangement (14) and the loudspeaker arrangement (15) are arranged on the connection channel (8) in such a way that the microphone arrangement (14) is closer to the input connection (2) than the loudspeaker arrangement (3),
wherein the microphone arrangement (14) has a directional characteristic, so that the microphone arrangement (14) registers sound coming from the side of the input connection (2) more strongly than sound coming from the side of the loudspeaker arrangement (15) and the output connection (3),
wherein the loudspeaker arrangement (15) has a directional characteristic, so that sound emitted by the loudspeaker arrangement (15) is conducted more strongly to the side of the output connection (3) than to the side of the microphone arrangement (14) and the input connection (2),
and wherein there is a closure device (9) which is switchable between an open position and a closed position, wherein the closure device (9) in the closed position closes the connection channel (8) between the microphone arrangement (14) and the loudspeaker arrangement (15), and in the open position the connection channel (8) between the microphone arrangement (14) and the loudspeaker arrangement (15) is open,
**characterized in**
**that** the connecting channel (8) between the input connection (2) and the output connection (3) runs along a longitudinal axis,
and in that the microphone arrangement (14) and the loudspeaker arrangement (15) comprise directional channels (14a, 15a) which branch off from the connecting channel (8) both in the open position and in the closed position of the closure device (9) and run inclined to the connecting channel (8).

2. Intermediate member (1) according to claim 1, **characterized in that**
the closure device (9) is designed to be manually mechanically switchable,
in particular wherein the closure device (9) has a latch for the closed position and a latch for the open position.

3. Intermediate member (1) according to claim 1 or claim 2, **characterized in that** the closure device (9) is designed having an iris diaphragm.

4. Intermediate member (1) according to claim 3, **characterized in that** the iris diaphragm is switchable between the closed position and the open position via a rotatable actuating ring (7).

5. Intermediate member (1) according to claim 4, **characterized in that**
the rotatable actuating ring (7) is designed having an actuating element (7a) with which the actuating ring (7) can be manually adjusted between a rotation position for a boost operation and a rotation position for a mechanical, non-boost operation,
in particular wherein the actuating element (7a) protrudes radially relative to the rest of the intermediate member (1).

6. Intermediate member (1) according to any of the preceding claims, **characterized in that** the input connection (2) and/or the output connection (3) are designed having
- a connection thread and/or
- a plug connection, in particular a conically shaped plug connection.

7. Intermediate member (1) according to any of the preceding claims, **characterized in that** the input connection (2) and/or the output connection (3) are designed in the form of a socket and/or in the form of a pipe socket.

8. Intermediate member according to claim 7, **characterized in that** the input connection (2) is designed in the form of a socket and the output connection (3) is designed in the form of a pipe socket.

9. Intermediate member (1) according to any of the preceding claims, **characterized in that** the electronic amplifier (16) comprises at least one filtering function which can be applied to the recorded signals, in particular
- a frequency filter, preferably a switchable frequency filter in order to adapt to different shapes of a stethoscope head (21) and/or a frequency filter having a plurality of individually adjustable frequency ranges, and/or
- a subtraction filter for subtracting ambient noise, in particular wherein the intermediate member (1) has at least one further microphone (19) for identifying the ambient noise to be subtracted, preferably wherein the at least one further microphone (19) is arranged on the outside of the intermediate member (1).

10. Intermediate member (1) according to any of the preceding claims, **characterized in that** the energy storage (17) is designed as a rechargeable energy storage (17), in particular wherein the intermediate member (1) comprises a wireless charging device (18) for the energy storage (17).

11. Intermediate member (1) according to any of the preceding claims, **characterized in that** the intermediate member (1) comprises
- at least one input device (4a-c) for setting the amplifier arrangement (5); and/or
- a display (6) for showing a patient noise, in particular a breathing noise or a heartbeat, and/or
- an electronic analysis device for evaluating a patient noise; and/or
- a display (6) for showing information derived from a patient noise, in particular a heart rate or a respiratory rate, and/or
- a display (6) for showing a remaining energy reserve of the energy storage (17) or for warning when a critical remaining energy reserve of the energy storage (17) is reached; and/or
- a display (6) for showing an amplification factor of the electronic amplifier arrangement (5); and/or
- a display (6) for showing a frequency filter setting; and/or
- a display (6) for showing an ambient noise compensation setting; and/or
- a wireless data transmission interface, in particular for WLAN (20) or Bluetooth; and/or
- a wired data transmission interface, in particular for USB,
- an on/off switch (4a) for the electronic amplification arrangement (5).

12. Intermediate member (1) according to any of the preceding claims, **characterized in that**
the intermediate member (1) is predominantly made of plastics material,
in particular of a sound-insulating plastics material.

13. Intermediate member (1) according to any of the preceding claims, **characterized in that** the intermediate member (1) has an outer casing made of plastics material, in particular of a sound-insulating and/or rubber-elastic plastics material.

14. Intermediate member (1) according to any of the preceding claims, **characterized in that**
the electronic amplifier (16) has at least one filter function that can be applied to the recorded signals, namely a subtraction filter for subtracting ambient noise, and the intermediate member (1) has at least one further microphone (19) for identifying the ambient noise to be subtracted, which further microphone (19) is arranged on the outside of the intermediate member (1).

15. Intermediate member (1) according to any of the preceding claims, **characterized in that** the directional channels (14a, 15a) run at an angle of approximately 45° and at an angle of approximately 135° with respect to the longitudinal axis of the connecting channel (8).

16. Stethoscope arrangement (46) comprising
- a mechanical stethoscope (40) having a stethoscope head (41), a sound transmission hose (42), two ear hooks (43a-b) and two ear interfaces (45a-b),
- at least one intermediate member (1) according to any of claims 1 to 15, wherein the at least one intermediate member (1) is installed in the mechanical stethoscope (40) between the stethoscope head (41) and the two ear interfaces (45a-b).

17. Use of an intermediate member (1) according to any of claims 1 to 15 for retrofitting a mechanical stethoscope (40),
wherein the intermediate member (1) is installed between a stethoscope head (41) and an ear interface (45a-b) of the stethoscope (40),
wherein a first stethoscope part (11) comprising the stethoscope head (41) is connected to the input connection (2) of the intermediate member (1), and a second stethoscope part (13) comprising the ear interface (45a-b) is connected to the output connection (3).

18. Use according to claim 17, **characterized in that** the intermediate member (1) is connected to the stethoscope head (41) at the input connection (2) with or without a connection adapter and to a sound transmission hose (42) at the outlet connection (3) with or without a connection adapter.

19. Use according to claim 17, **characterized in that** the intermediate member (1) is connected to one end of an ear hook (43a-b) at the input connection (2) with or without a connection adapter and to an earpiece (44a-b) at the output connection (3) with or without a connection adapter.

## Revendications

1. Pièce intercalaire (1) destinée à un stéthoscope (40), ladite pièce intercalaire (1) comprenant
- un raccord d'entrée (2), affecté à une première partie (11) du stéthoscope qui achemine du son provenant d'un pavillon (41) dudit stéthoscope,
- un raccord de sortie (3), affecté à une seconde partie (13) du stéthoscope qui canalise du son vers au moins un interface auriculaire (45a-b),
- entre le raccord d'entrée (2) et le raccord de sortie (3), un canal de liaison (8) apte à conduire du son vers ledit raccord de sortie (3) à partir dudit raccord d'entrée (2), et
- un dispositif (5) d'amplification électronique incluant
a) un ensemble (14) de microphones, conçu pour saisir du son capté par l'intermédiaire du raccord d'entrée (2),
b) un ensemble (15) de haut-parleurs, conçu pour délivrer du son au raccord de sortie (3),
c) un amplificateur électronique (16) à l'aide duquel des signaux saisis par ledit ensemble (14) de microphones peuvent être transmis, avec amplification, audit ensemble (15) de haut-parleurs,
d) un accumulateur d'énergie (17), conçu pour alimenter ledit dispositif (5) d'amplification électronique en courant électrique ;
sachant que l'ensemble (14) de microphones et l'ensemble (3) de haut-parleurs sont implantés, sur le canal de liaison (8), de façon telle que ledit ensemble (14) de microphones soit plus rapproché du raccord d'entrée (2) que ledit ensemble (15) de haut-parleurs,
l'ensemble (14) de microphones présentant une caractéristique directionnelle telle que ledit ensemble (14) de microphones enregistre du son, émanant du côté du raccord d'entrée (2), plus intensément que du son émanant du côté de l'ensemble (15) de haut-parleurs et du raccord de sortie (3),
l'ensemble (15) de haut-parleurs présentant une caractéristique directionnelle telle que du son émis par ledit ensemble (15) de haut-parleurs soit dirigé, vers le côté du raccord de sortie (3), plus intensément que du côté de l'ensemble (14) de microphones et du raccord d'entrée (2),
et sachant qu'il est prévu un système d'occultation (9) pouvant être commuté entre une position ouverte et une position fermée,
ledit système d'occultation (9) obturant le canal de liaison (8) entre l'ensemble (14) de microphones et l'ensemble (15) de haut-parleurs, dans ladite position fermée, et ledit canal de liaison (8) étant ouvert entre ledit ensemble (14) de microphones et ledit ensemble (15) de haut-parleurs, dans ladite position ouverte,
**caractérisée par le fait**
**que** le canal de liaison (8) s'étend le long d'un axe longitudinal, entre le raccord d'entrée (2) et le raccord de sortie (3) ;
et par le fait que des canaux directeurs (14a, 15a), faisant partie de l'ensemble (14) de microphones et de l'ensemble (15) de haut-parleurs, bifurquent du canal de liaison (8) tant dans la position ouverte, que dans la position fermée du système d'occultation (9), et s'étendent à l'oblique par rapport audit canal de liaison (8).

2. Pièce intercalaire (1) selon la revendication 1,
**caractérisée par le fait**
**que** le système d'occultation (9) est réalisé avec faculté de commutation mécanique manuelle,
ledit système d'occultation (9) étant pourvu d'un dispositif d'encliquetage dédié à la position fermée, et d'un dispositif d'encliquetage dédié à la position ouverte.

3. Pièce intercalaire (1) selon la revendication 1 ou 2,
**caractérisée par le fait**
**que** le système d'occultation (9) est équipé d'un diaphragme à iris.

4. Pièce intercalaire (1) selon la revendication 3,
**caractérisée par le fait**
**que** le diaphragme à iris peut être commuté entre la position fermée et la position ouverte, par l'intermédiaire d'une bague rotative d'actionnement (7).

5. Pièce intercalaire (1) selon la revendication 4,
**caractérisée par le fait**
**que** la bague rotative d'actionnement (7) est dotée d'un élément d'actionnement (7a) par lequel ladite bague d'actionnement (7) peut être déplacée manuellement entre un emplacement pris par rotation, affecté à un mode amplification, et un emplacement pris par rotation et affecté à un mode mécanique non amplifié,
lequel élément d'actionnement (7a) fait notamment saillie, dans le sens radial, par rapport au reste de ladite pièce intercalaire (1).

6. Pièce intercalaire (1) selon l'une des revendications précédentes, **caractérisée par le fait**
**que** le raccord d'entrée (2) et/ou le raccord de sortie (3) est (sont) pourvu(s)
- d'un filetage de raccordement et/ou
- d'une jonction enfichable, en particulier d'une jonction enfichable de configuration conique.

7. Pièce intercalaire (1) selon l'une des revendications précédentes,
**caractérisée par le fait**
**que** le raccord d'entrée (2) et/ou le raccord de sortie (3) est (sont) réalisé(s) en forme de douille et/ou en forme de manchon tubulaire.

8. Pièce intercalaire (1) selon la revendication 7,
**caractérisée par le fait**
**que** le raccord d'entrée (2) et le raccord de sortie (3) sont réalisés, respectivement, en forme de douille et en forme de manchon tubulaire.

9. Pièce intercalaire (1) selon l'une des revendications précédentes,
**caractérisée par le fait**
**que** l'amplificateur électronique (16) présente au moins une fonction de filtrage pouvant être appliquée aux signaux saisis, en particulier
- un filtre de fréquence, de préférence un filtre de fréquence commutable, dévolu à l'adaptation à différentes formes d'un pavillon (21) de stéthoscope, et/ou un filtre de fréquence comptant plusieurs plages de fréquences pouvant être régulées individuellement, et/ou
- un filtre soustracteur dévolu à la soustraction de bruits environnants,
sachant notamment que ladite pièce intercalaire (1) est équipée d'au moins un microphone supplémentaire (19), en vue de déterminer des bruits environnants à soustraire, lequel microphone supplémentaire (19), à présence minimale, est de préférence disposé extérieurement sur ladite pièce intercalaire (1).

10. Pièce intercalaire (1) selon l'une des revendications précédentes,
**caractérisée par le fait**
**que** l'accumulateur d'énergie (17) est réalisé en tant qu'accumulateur d'énergie (17) rechargeable, ladite pièce intercalaire (1) étant équipée, en particulier, d'un chargeur (18) exempt de câblage et destiné audit accumulateur d'énergie (17).

11. Pièce intercalaire (1) selon l'une des revendications précédentes,
**caractérisée par le fait**
**que** ladite pièce intercalaire (1) comporte
- au moins un dispositif de saisie (4a-c), affecté au réglage du dispositif d'amplification (5) ; et/ou
- un affichage (6) affecté à la visualisation d'un bruit produit par un patient, en particulier d'un bruit de respiration ou d'un battement cardiaque, et/ou
- un système d'analyse électronique, affecté à l'évaluation d'un bruit produit par un patient ; et/ou
- un affichage (6) affecté à la visualisation d'une information dérivée d'un bruit produit par un patient, en particulier d'une fréquence cardiaque ou d'une fréquence respiratoire, et/ou
- un affichage (6) conçu pour visualiser une réserve en énergie restante de l'accumulateur d'énergie (17), ou pour alerter lorsqu'est atteinte une réserve critique en énergie restante dudit accumulateur d'énergie (17) ; et/ou
- un affichage (6) affecté à la visualisation d'un facteur d'amplification du dispositif (5) d'amplification électronique ; et/ou
- un affichage (6) affecté à la visualisation d'un réglage du filtre de fréquence ;
et/ou
- un affichage (6) affecté à la visualisation d'un réglage d'une compensation de bruits environnants ; et/ou
- un interface de transmission de données exempt de câblage, notamment à destination *WLAN* (20) ou *Bluetooth* ; et/ou
- un interface de transmission filaire de données, notamment à destination *USB,*
- un interrupteur (4a) marche/arrêt, dédié audit dispositif (5) d'amplification électronique.

12. Pièce intercalaire (1) selon l'une des revendications précédentes,
**caractérisée par le fait**
**que** ladite pièce intercalaire (1) est majoritairement fabriquée en matière plastique, notamment en une matière plastique insonorisante.

13. Pièce intercalaire (1) selon l'une des revendications précédentes,
**caractérisée par le fait**
**que** ladite pièce intercalaire (1) est munie d'une enveloppe extérieure en matière plastique, notamment en une matière plastique insonorisante et/ou douée de l'élasticité du caoutchouc.

14. Pièce intercalaire (1) selon l'une des revendications précédentes,
**caractérisée par le fait**
**que** l'amplificateur électronique (16) présente au moins une fonction de filtrage pouvant être appliquée aux signaux saisis, à savoir un filtre soustracteur dévolu à la soustraction de bruits environnants, et ladite pièce intercalaire (1) est équipée, en vue de déterminer des bruits environnants à soustraire, d'au moins un microphone supplémentaire (19) disposé extérieurement sur ladite pièce intercalaire (1).

15. Pièce intercalaire (1) selon l'une des revendications précédentes,
**caractérisée par le fait**
**que** les canaux directeurs (14a, 15a) s'étendent suivant un angle d'environ 45°, et suivant un angle d'environ 135°, par rapport à l'axe longitudinal du canal de liaison (8).

16. Agencement stéthoscopique (46) comprenant
- un stéthoscope mécanique (40) muni d'un pavillon (41), d'un tuyau souple (42) transmetteur de sons, de deux tubes auriculaires (43a-b) et de deux interfaces auriculaires (45a-b),
- au moins une pièce intercalaire (1) conforme à l'une des revendications 1 à 15, laquelle pièce intercalaire (1) à présence minimale est intégrée, dans le stéthoscope mécanique (40), entre le pavillon (41) dudit stéthoscope et les deux interfaces auriculaires (45a-b).

17. Utilisation d'une pièce intercalaire (1) conforme à l'une des revendications 1 à 15, en vue du remaniement structurel d'un stéthoscope mécanique (40),
ladite pièce intercalaire (1) étant intégrée entre un pavillon (41) et un interface auriculaire (45a-b) dudit stéthoscope (40),
sachant qu'une première partie (11) du stéthoscope, incluant ledit pavillon (41) du stéthoscope, est rattachée au raccord d'entrée (2) de la pièce intercalaire (1) et qu'une seconde partie (13) dudit stéthoscope, incluant l'interface auriculaire (45a-b), est rattachée au raccord de sortie (3).

18. Utilisation selon la revendication 17,
**caractérisée par le fait**
**que** la pièce intercalaire (1) est rattachée au pavillon (41) du stéthoscope au niveau du raccord d'entrée (2), avec ou sans adaptateur de raccordement, et à un tuyau souple (42) transmetteur de sons, au niveau du raccord de sortie (3), avec ou sans adaptateur de raccordement.

19. Utilisation selon la revendication 17,
**caractérisée par le fait**
**que** la pièce intercalaire (1) est rattachée à une extrémité d'un tube auriculaire (43a-b) au niveau du raccord d'entrée (2), avec ou sans adaptateur de raccordement, et à un embout auriculaire (44a-b), au niveau du raccord de sortie (3), avec ou sans adaptateur de raccordement.
